# EUROPÄISCHE PATENTSCHRIFT

(11) **EP 3 764 946 B1**
(45) Veröffentlichungstag und Bekanntmachung des Hinweises auf die Patenterteilung: **03.06.2026**
(21) Anmeldenummer: 19715817.3
(22) Anmeldetag: 14.03.2019
(51) Int. Cl.: A61F 2/07, A61F 2/915, A61F 2/852

(54) **DOPPELSTENT**
DOUBLE STENT
STENT DOUBLE

(30) Priorität: 14.03.2018 DE 102018105925
(43) Veröffentlichungstag der Anmeldung: 20.01.2021
(73) Patentinhaber: Bentley InnoMed GmbH, 72379 Hechingen (DE)
(72) Erfinder: NEUß, Malte, 53121 Bonn (DE)
(74) Vertreter: Hoefer & Partner Patentanwälte mbB
(86) Internationale Anmeldenummer: PCT/EP2019/056453
(87) Internationale Veröffentlichungsnummer: WO 2019/175330

(56) Entgegenhaltungen:
- WO-A1-2017/125312
- WO-A1-2018/078019

## Beschreibung

Die Erfindung betrifft einen Doppelstent mit zwei koaxial angeordneten Stents, wobei zwischen einem ersten inneren Stent und einem zweiten äußeren Stent eine erste Membran angeordnet ist und auf dem zweiten Stent eine zweite Membran, wobei die Membranenden der ersten und der zweiten Membran an den Enden des Stents zusammengefasst sind und auf die Innenseite des ersten Stents umgeschlagen und dort fixiert sind.

Der Doppelstent dient insbesondere als Stentgraft zur Überbrückung von vaskulären Fehlbildungen, etwa Aneurysmen und Shunts, aber auch zur Verstärkung von labilen, brüchigen oder thrombotischen Gefäßwänden. Er wird ferner eingesetzt als Überbrückung bei Abzweigungen aus gestenteten Gefäßen oder Prothesen.

Stentgrafts zur Überbrückung von vaskulären Fehlbildungen sind in einer Vielzahl von Formen bekannt. In der Regel bestehen sie aus einem Stent, der mit einer Membran ganz oder teilweise überzogen ist. Die Membran schließt die vaskuläre Fehlbildung gegen das Gefäß ab, der Stent hält das Gefäß offen und sorgt dafür, dass die Membran eng an der Gefäßwand anliegt.

Ein Problem bei Stentgrafts ist die Verankerung der Membran am Stent. Hierzu wurden Doppelstents entwickelt, bei denen die Membran zwischen einem äußeren und einem inneren Stent gehalten wird. Bei der Expansion eines solchen Doppelstents nimmt die Membran an der radialen Erweiterung teil, bleibt aber zwischen den beiden Stents eingeklemmt.

Ein solcher Doppelstent ist beispielsweise aus der DE 197 20 115 A1 bekannt. Der dort beschriebene Stent hat sich an und für sich bewährt, ist aber in zweierlei Hinsicht verbesserungsfähig.

Zum einen ergeben sich häufig Probleme mit der Dichtigkeit, da die Membran nicht unmittelbar an der Gefäßwand anliegt und/oder bei der Expansion des Doppelstents beschädigt wird. In beiden Fällen wird der Doppelstent den an ihn gestellten Anforderungen, nämlich der Abschottung beispielsweise einer vaskulären Fehlbildung, nicht gerecht.

Zum anderen kann es bei der Expansion des Doppelstents dazu kommen, dass der Verbund aus zwei Stents und einer Membran an Zusammenhalt verliert, beispielsweise wenn die beiden Stents ein unterschiedliches Expansionsverhalten - etwa aufgrund örtlicher Gegebenheiten - haben.

Doppelstents, die aus der Kombination zweier ballonexpandierbarer Stents bestehen, weisen in der Regel eine hohe Radialkraft auf, die zu einer zuverlässigen Platzierung und Fixierung der Membranen führt. Allerdings geht die hohe Radialkraft mit einem Verlust an Flexibilität einher. Zudem werden für die Expansion erhebliche Drücke benötigt. Die Wandstärke eines solchen mit Membranen versehenden Doppelstent sollte sich in Grenzen halten, um das Gefäßvolumen nicht unnötig einzuengen, ohne dass dies aber zu Lasten der Funktionalität geht.

Die WO 2017/125312 A1 beschreibt einen weiteren Doppelstent, der neben einem inneren und einem äußeren Stent auch eine innere und eine äußere Membran aufweist, die sich jeweils gegenseitig ergänzen.

Der Erfindung liegt damit die Aufgabe zu Grunde, einen Doppelstent bereitzustellen, der den Anforderungen an die Dichtigkeit und Zuverlässigkeit genügt, den notwendigen Zusammenhalt gewährleistet und eine hinreichend hohe Radialkraft aufweist. Gleichzeitig sollte der Stent eine hinreichende Flexibilität aufweisen.

Diese Aufgabe wird mit einem Doppelstent gemäß Anspruch 1 gelöst, bei dem die beiden Stents aus unterschiedlichen Materialien bestehen und sich in Länge und Radius unterscheiden
Im erfindungsgemäßen Doppelstent unterscheiden sich der innere und der äußere Stent in den Eigenschaften und Materialien. Ein Stent ist dabei ein ballonexpandierbarer Stent, der die benötigte Radialkraft bereitstellt, der andere Stent besteht aus einem anderem Material mit geringerer Radialkraft, beispielsweise aus einem biologisch abbaubaren Material oder aus einer Formgedächtnislegierung, die selbstexpandierend ist. Derartige Materialien sind bekannt und vielfach beschrieben. Der Stent mit der geringeren Radialkraft ist flexibler, d. h., er kann eine geringere Wandstärke aufweisen. Er dient insbesondere der Fixierung der ersten und/oder zweiten Membran.

Der ballonexpandierbare Stent mit hoher Radialkraft ist insbesondere ein Stent aus einer Kobalt-Chrom-Legierung wie er herkömmlicherweise als Gefäßstütze eingesetzt wird. Dieser ballonexpandierbare Stent kann sowohl innen als auch außen zur Gefäßwand zeigend eingesetzt werden, also als erster innerer oder zweiter äußerer Stent dienen. Wird er als erster innerer Stent eingesetzt, dient er auch der Fixierung der Membranenden. Ist er als zweiter äußerer Stent eingesetzt, sorgt er für den notwendigen Anpressdruck, der zum einen der Fixierung des Doppelstents an der Gefäßwand dient, gegebenenfalls auch der Erweiterung des Gefäßes, zum anderen aber auch der Fixierung der äußeren Membranen an der Gefäßwand.

Der andere Stent mit der geringeren Radialkraft kann beispielsweise ein biologisch abbaubarer Stent sein, wie er üblicherweise aus biologisch abbaubaren Kunststoffen (Polylactid und/oder - galactid) gefertigt wird und vielfach beschrieben ist. Biologisch abbaubare Metallstents, beispielsweise aus Magnesiumlegierungen, kommen hier ebenfalls in Frage. Auch diese Stents sind vielfach beschrieben. Ein solcher Stent kommt nur als zweiter äußerer Stent in Frage und dient der Fixierung der äußeren Membranen an der Gefäßwand und der inneren Membran im Stentverbund.

Alternativ kann der Stent mit der geringeren Radialkraft aus einer Nickel-Titan-Legierung bestehen, etwa Nitinol. Ein solcher Stent kommt sowohl als erster innerer als auch als zweiter äußerer Stent in Frage. Als erster innerer Stent sorgt er für die Fixierung der Membranenden an und liefert den nötigen Anpressdruck für die inneren Membranen an den zweiten äußeren Stent. Als zweiter äußerer Stent sorgt er in der Expansionsphase für eine schnelle Fixierung der äußeren Membranen an der Gefäßwand und für einen guten Sitz der inneren Membran zwischen den beiden Stents des Verbunds.

Bevorzugt ist die Kombination eines Stents aus einer Kobalt-Chrom-Legierung mit einem selbstexpandierenden Stent aus einer Nickel-Titan-Legierung, wobei letzterer besonders bevorzugt den ersten inneren Stent des Doppelstentverbundes bildet.

In diesem Fall ist zweckmäßig der innere erste Stent um 5 bis 10 mm, vorzugsweise um 8 mm länger als der äußere zweite Stent und hat in seinem durch Formgedächtnis festgelegten Endzustand einen um 1 bis 6 mm, vorzugsweise 3 mm größeren Durchmesser als der äußere zweite Stent. Durch dieses Übermaß im Durchmesser wird sichergestellt, dass im Endzustand die innere Membran stets ausreichend fest zwischen dem inneren Stent und dem äußeren Stent eingespannt bleibt.

Zur besseren Verankerung des Doppelstents in einem Gefäß oder einer Prothese kann der innere erste Stent zusätzlich mit radial vorstehenden Widerhaken oder einer trompetenartigen Ausformung versehen sein, die erforderlichenfalls mit ePTFE überzogen sein können.

Der erfindungsgemäße Doppelstent weist nicht nur einen inneren und einen äußeren Stent auf sondern auch eine innere und äußere Membran. Dabei ergänzen sich die beiden Membranen hinsichtlich der Dichtigkeit. Die äußere zweite Membran dient als Schutz und Ergänzung der inneren ersten Membran; wenn die innere Membran bei der Expansion beschädigt wird, beispielsweise einreißt; ist die äußere Membran in der Lage, diesen Defekt auszugleichen und umgekehrt. Des Weiteren hält die äußere Membran das Konstrukt zusammen, wobei Verankerung der Enden der äußeren Membran - zusammen mit den Enden der inneren Membran - auf der Innenseite des inneren Stent zu diesem Zusammenhalt beiträgt.

Für die erfindungsgemäß eingesetzten Stents kommen übliche Stentdesigns infrage, wie sie vielfach bei ballonexpandierbaren und selbst expandierenden Stents entwickelt wurden. Für ballonexpandierbare Stents kommen übliche Materialien in Frage, beispielsweise Stahllegierungen für den medizinischen Gebrauch, Kobalt-Chrom-Legierungen und dergleichen. Für selbst expandierende Stents kommen insbesondere Materialien mit einem Formgedächtnis infrage, etwa Nickel-Titan-Legierungen.

Die Stents werden in der Regel aus einem Rohr mit geeignetem Durchmesser mithilfe eines Laserstrahls geschnitten. Sie verfügen über eine Maschenstruktur mit Ringsegmenten und Verbindungsstegen zwischen den Ringsegmenten.

Die Stents können beispielsweise eine Maschenstruktur haben, wie sie von einander kreuzenden Stegen gebildet wird. Bevorzugt sind Stents, die aus einer Mehrzahl von mäandrierenden Ringsegmenten gebildet werden, wobei die Ringsegmente durch Verbindungsstege mit benachbarten Ringsegmenten verbunden sind. Auch in diesem Fall entstehen Maschen, deren Größe durch die Häufigkeit der Verbindungsstege zwischen zwei benachbarten Ringsegmenten bestimmt ist. Eine solche Stentstruktur ist geeignet, die bei der Expansion auftretende Längenreduktion zumindest teilweise auszugleichen, je nach Anordnung und Form der Verbindungsstege.

Zur Fixierung der Membranen an der Innenseite des inneren Stents können verschiedene Methoden herangezogen werden. Zum einen können die Membranen vernäht oder verklebt werden. Bevorzugt ist das Verschweißen, beispielsweise mit Ultraschall, oder das Verschmelzen, auch durch die Maschen des inneren Stents hindurch, wenn dieser bei der Herstellung einer ersten Dilation unterzogen wird. Der fertige Doppelstent wird dann anschließend auf einen Ballon gekrimpt.

Die Membranen am inneren ersten Stent können auch festgeklemmt sein, beispielsweise an dort vorhandenen Federzungen. Die Federzungen weisen dabei stentauswärts, d. h., sie weisen zum Stentrand. Solche Federzungen können beispielsweise auswärtsweisende Membranbögen der Ringsegmente sein, wobei die Folienenden zwischen den Membranbögen und vom gleichen Ringsegment ausgehenden Verbindungsstegen festgeklemmt werden (WO 2012/084202 A2).

Das Festklemmen der Membranenden auf der Innenseite des inneren Stent führt zu einer zuverlässigen Verankerung der beiden Membranen und stärkt den Verbund aus innerem Stent, innerer Membran, äußerem Stent und äußerer Membran.

Für die Membranen kann jedes dafür geeignete biologische oder künstliche Material verwandt werden. In der Regel bestehen die Membranen aus einem Kunststoff, vorzugsweise einem Kunststoffschlauch, der über den jeweiligen Stent gezogen ist. Ein geeignetes Material ist beispielsweise Polytetrafluorethylen, PTFE, insbesondere ePTFE, dass die benötigte Elastizität für die Expansion aufweist. Andere medizinisch geeignete Kunststoffe, etwa Polyester, Polyolefine, Polyurethane, Polyurethancarbonat und dergleichen, können ebenfalls eingesetzt werden.

Es versteht sich, dass für den inneren und den äußeren Stent unterschiedliche Designs verwandt werden können und die innere und äußere Membran aus verschiedenen Materialien gefertigt sein können.

Die Verwendung von zwei Stents und zwei Membranen führt naturgemäß zu einer relativ hohen Wandstärke des Konstrukts, was die Manövrierfähigkeit im Gefäßsystem eines Patienten einschränkt. Dem kann dadurch begegnet werden, dass die Wandstärke der zum Schneiden der Stents verwandten Rohre, insbesondere für den Stent mit geringerer Radialkraft, gering gewählt wird, beispielsweise im Bereich von 0,05 bis 0,50 mm, vorzugsweise 0,10 bis 0,20 mm und insbesondere etwa 0,15 mm. Auch die Stegbreite kann vermindert werden auf beispielsweise 0,05 bis 0,50 mm, vorzugsweise 0,10 bis 0,20 mm und insbesondere etwa 0,15 mm.

Es ist ferner bevorzugt, den äußeren Stent mit kleineren Maschen zu versehen als den inneren Stent. Hierdurch wird bei der Expansion eine Pressspannung erzeugt, die sich vorteilhaft auf die Radialkraft und den Zusammenhalt des Konstrukts auswirkt. Erzielt wird eine hohe Festigkeit und Dauerhaftigkeit des Konstrukts.

Der erfindungsgemäße Doppelstent ist insbesondere geeignet, in Abzweige aus gestenteten Gefäßen eingesetzt zu werden und den sich dabei zwischen gestentetem Gefäß und Abzweigung bildenden Raum zu überbrücken.

Die Erfindung wird durch die beiliegenden Abbildungen näher erläutert, die bevorzugte Ausführungsformen der Erfindung zeigen. Es versteht sich, dass in den Abbildungen gezeigten Merkmale jeweils für sich Teil der Erfindung sind und nicht nur im Zusammenhang mit anderen Merkmalen der Abbildung zu verstehen sind. Es zeigen:
- Figur 1:: schematisch einen Längsschnitt durch den Endbereich der Wandung eines Doppelstents gemäß der Erfindung;
- Figur 2:: eine Draufsicht auf die Abwicklung der Wandung des inneren Stents in dessen Endbereich;
- Figur 3:: eine Variante zu Figur 2.

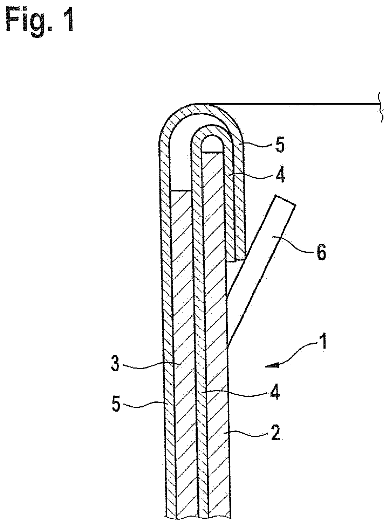

Der in Figur 1 insgesamt mit dem Bezugszeichen 1 bezeichnete Doppelstent gemäß der Erfindung weist einen ersten inneren Stent 2 und einen zweiten äußeren Stent 3 auf, die koaxial zueinander angeordnet sind. Der äußere Stent 3 ist etwas kürzer als der innere Stent 2. Der gesamte Doppelstent 1 ist im nicht expandierten Zustand dargestellt. Zwischen dem inneren Stent 2 und dem äußeren Stent 3 befindet sich eine erste innere Membran 4. Der äußere Stent 3 ist von einer zweiten äußeren Membran 5 umgeben. Beide Membranen 4 und 5 bestehen aus ePTFE.

Die innere Membran 4 und die äußere Membran 5 sind an ihren Enden zusammengefasst und um den oberen Rand des Doppelstents 1 nach innen in den Hohlraum des inneren Stents 2 umgeschlagen. Beispielhaft dargestellt ist eine von mehreren Federzungen 6, die Bestandteil des inneren Stents 2 sind, nach innen abgebogen sind und zum Festklemmen der nach innen umgeschlagenen Ränder der Membranen 4 und 5 dienen.

Der aus Figur 1 hervorgehende innere Stent 2 besteht zweckmäßig aus einer geeigneten Formgedächtnislegierung, z. B. aus Nickel-Titan (Nitinol), und hat proximal eine Überlänge von 5 mm bis 10 mm, vorzugsweise von 8 mm. Außerdem hat er in seinem durch das Formgedächtnis festgelegten Endzustand einen Durchmesser, der um 1 bis 6 mm, vorzugsweise um 3 mm größer ist als der Durchmesser des äußeren Stents 3. Hierdurch wird sichergestellt, dass die innere Membran 4 stets ausreichend fest zwischen dem inneren Stent 2 und dem äußeren Stent 3 eingeklemmt bleibt. Zur besseren Verankerung in einem Gefäß oder einer Prothese kann der Stent 2 zusätzlich mit radial vorstehenden Widerhaken oder einer trompetenförmigen Ausformung versehen sein, die erforderlichenfalls mit ePTFE überzogen sein können.

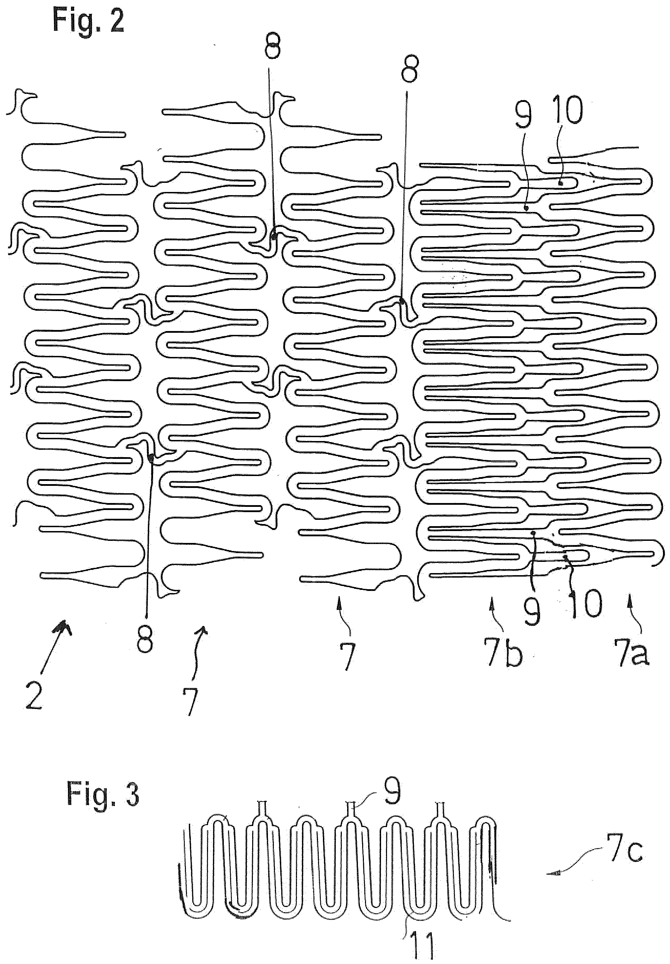

Figur 2 zeigt das Design der Wandung des inneren Stents 2 in dessen auch in Figur 1 dargestelltem Endbereich, und zwar mit einer konkreten Ausgestaltung der in Figur 1 nur schematisch dargestellten Federzungen 6.

Wie aus Figur 2 hervorgeht, besteht die Wandung des inneren Stents 2 im Längenbereich des Stents 2 aus einer Vielzahl von Ringsegmenten 7, von denen jedes ein umlaufendes, mäanderförmiges Band aufweist. Diese mäanderförmigen Bänder sind axial durch in axialer Richtung nachgiebige Verbindungsstege miteinander verbunden. Insoweit entspricht der Wandaufbau des inneren Stents 2 dem weit verbreiteten üblichen Aufbau von Stents.

Im Gegensatz zu den üblichen Stents weist der Stent 2 gemäß Figur 2 im Endbereich zwei abweichend ausgestaltete Ringsegmente 7a und 7b auf, um die oben in Figur 1 mit dem Bezugszeichen 6 bezeichneten Federzungen des ersten inneren Stents 2 zur Verfügung zu stellen. Dabei bildet das Ringsegment 7a den abschließenden Außenrand des Stents 2, während das Ringsegment 7b zwischen dem Außenrand und dem Längenbereich des Stents 2 angeordnet ist, siehe DE 10 2015 106 052 A1.

Die beiden Ringsegmente 7a und 7b weisen ebenfalls jeweils ein umlaufendes, mäanderförmiges Band auf. Dabei sind die zum Längenbereich des Stents 2 weisenden Mäanderbögen beider Ringsegmente 7a und 7b durch axial verlaufende Verbindungsstege 9 miteinander verbunden. Demgegenüber sind die zum Ende des Stents 2 weisenden Mäanderbögen der beiden Ringsegmente 7a und 7b nicht miteinander verbunden. Stattdessen sind die zum Ende des Stents 2 weisenden Mäanderbögen des Ringelementes 7b mit Blindstegen 10 versehen, die in die in der gleichen Richtung ausgerichteten Mäanderbögen des Ringsegmentes 7a hineinragen, ohne mit diesen verbunden zu sein. Diese Blindstege 10 bilden zusammen mit den sie tragenden Mäanderbögen des Ringsegmentes 7b die in Figur 1 nur schematisch dargestellten und dort mit dem Bezugszeichen 6 bezeichneten Federzungen.

Die Figur 3 zeigt eine weitere Möglichkeit zur Ausbildung der oben diskutierten Federzungen, die in Figur 1 mit dem Bezugszeichen 6 bezeichnet sind. Hierzu ist endseitig an dem inneren Stent 2 ein Ringsegment 7c vorgesehen, welches ebenfalls ein umlaufendes, mäanderförmiges Band aufweist dessen zum Ende des Stents 2 weisenden Mäanderbögen über ihre Länge mit Einschnitten 11 versehen sind und auf diese Weise elastisch verformbare Federzungen bilden, die zwischen sich - nach Art von Büroklammern - die in Figur 1 dargestellten Enden der Membranen 4 und 5 festklemmen können, siehe WO 2012/084202 A2.

## Patentansprüche

1. Doppelstent mit zwei koaxial angeordneten Stents (2, 3), wobei zwischen einem ersten inneren Stent (2) und dem zweiten äußeren Stent (3) eine erste Membran (4) angeordnet ist und auf dem zweiten Stent (3) eine zweite Membran (5), wobei die Membranenden der ersten und der zweiten Membran (4, 5) an den Enden der Stents (2, 3) zusammengefasst und auf die Innenseite des ersten Stents (2) umgeschlagen und dort fixiert sind, wobei der erste innere Stent (2) aus einem ersten Material gefertigt ist und der zweite äußere Stent (3) aus einem zweiten Material, **dadurch gekennzeichnet, dass** der äußere zweite Stent (3) aus einer Kobalt-Chrom-Legierung besteht und der innere erste Stent (2) aus einer Formgedächtnislegierung besteht und der innere erste Stent (2) um 5 bis 10 mm länger als der äußere zweite Stent (3) ist und in seinem durch Formgedächtnis festgelegten Endzustand einen um 1 bis 6 mm größeren Durchmesser als der äußere zweite Stent (3) hat.

2. Doppelstent nach Anspruch 1, **dadurch gekennzeichnet, dass** der innere erste Stent (2) aus einer Nickel-Titan-Legierung besteht.

3. Doppelstent nach Anspruch 1 oder 2, **dadurch gekennzeichnet, dass** der innere erste Stent (2) um 8 mm länger als der äußere zweite Stent (3) ist.

4. Doppelstent nach einem der Ansprüche 1 bis 3, **dadurch gekennzeichnet, dass** der innere erste Stent (2) in seinem durch Formgedächtnis festgelegten Endzustand einen um 3 mm größeren Durchmesser als der äußere zweite Stent (3) hat.

5. Doppelstent nach einem der Ansprüche 1 bis 4, **dadurch gekennzeichnet, dass** der innere erste Stent (2) an seinem proximalen Ende mit zusätzlichen Fixierelementen versehen ist.

6. Doppelstent nach Anspruch 5, **dadurch gekennzeichnet, dass** die Fixierelemente in Form von Widerhaken und/oder einer trompetenförmigen Aufweitung ausgestaltet sind.

7. Doppelstent nach einem der vorstehenden Ansprüche, **dadurch gekennzeichnet, dass** wenigstens der erste innere Stent (2) eine Mehrzahl von nebeneinander angeordneten Ringsegmenten (7, 7a, 7b) mit mäandrierender Struktur aufweist, die durch Verbindungsstege (8, 9) miteinander verbunden sind.

8. Doppelstent nach einem der vorstehenden Ansprüche, **dadurch gekennzeichnet, dass** die Membranen (4, 5) durch Verkleben, Vernähen, Verschweißen oder Festklemmen fixiert sind.

9. Doppelstent nach einem der Ansprüche 1 bis 8, **dadurch gekennzeichnet, dass** die Membranen (4, 5) in Federzungen (6) von Einschnitten in Mäanderbögen des ersten Stents (2) festgeklemmt sind.

10. Doppelstent nach Anspruch 9, **dadurch gekennzeichnet, dass** die Federzungen (6) stentauswärts weisen.

11. Doppelstent nach Anspruch 9 oder 10, **dadurch gekennzeichnet, dass** die Federzungen (6) in den Randbereichen des ersten Stents (2) angeordnet sind.

12. Doppelstent nach Anspruch 11, **dadurch gekennzeichnet, dass** die Federzungen (6) an Ringsegmenten (7b) ausgebildet sind, die benachbart zu randständigen Ringsegmenten (7a) angeordnet sind.

13. Doppelstent nach einem der Ansprüche 7 bis 12, **dadurch gekennzeichnet, dass** die Verbindungsstege (8) des ersten inneren Stents (2) und zweiten äußeren Stents (3) auf Lücke stehen.

14. Doppelstent nach einem der vorstehenden Ansprüche, **dadurch gekennzeichnet, dass** die erste (4) und/oder die zweite (5) Membran (4, 5) aus Kunststoff besteht.

15. Doppelstent nach Anspruch 14, **dadurch gekennzeichnet, dass** die erste (4) und/oder zweite (5) Membran (4, 5) aus PTFE besteht.

## Claims

1. Double stent comprising two coaxially arranged stents (2, 3), wherein a first membrane (4) is arranged between a first inner stent (2) and the second outer stent (3) and a second membrane (5) is arranged on the second stent (3), wherein the membrane ends of the first and the second membrane (4, 5) are brought together at the ends of the stents (2, 3) and are folded over onto the inner side of the first stent (2) and secured/fixed there, wherein the first inner stent (2) is made of a first material and the second outer stent (3) is made of a second material, **characterized in that** the second outer stent (3) consists of a cobalt-chromium alloy and the first inner stent (2) consists of a shape-memory alloy and **in that** the first inner stent (2) is 5 to 10 mm longer than the second outer stent (3) and, in its final form as determined by its shape memory characteristics, has a diameter 1 to 6 mm larger than the diameter of the second outer stent (3).

2. Double stent according to claim 1, **characterized in that** the first inner stent (2) consist of a nickel-titanium alloy.

3. Double stent according to claim 1 or 2, **characterized in that** the first inner stent (2) is 8 mm longer that the second outer stent (3).

4. Double stent according to any one of claims 1 to 3, **characterized in that** the first inner stent (2), in its final form as determined by its shape memory characteristics, has a diameter 3 mm larger than the diameter of the second outer stent (3)

5. Double stent according to any one of claims 1 to 4, **characterized in that** the first inner stent (2) is provided at its proximal end with additional fixation elements.

6. Double stent according to claim 5, **characterized in that** the fixation elements are in the form of barbs and/or a trumpet-shaped widening.

7. Double stent according to any one of the preceding claims, **characterized in that** at least the first inner stent (2) is provided with a plurality of ring segments (7, 7a, 7b) arranged side by side and having a meandering structure, said ring segments being connected to one another by means of connecting webs (8, 9).

8. Double stent according to any one of the preceding claims, **characterized in that** the membranes (4, 5) are secured/fixed by gluing/bonding, sewing, welding or clamping.

9. Double stent according to any one of claims 1 to 8, **characterized in that** the membranes (4, 5) are clamped in place in or between the flexible tongues (6) formed by applying incisions in the meandering arches of the first stent (2).

10. Double stent according to claim 9, **characterized in that** the flexible tongues (6) point to the outside of the stent.

11. Double stent according to claim 9 or 10, **characterized in that** the flexible tongues (6) are arranged in the peripheral regions of the first stent (2).

12. Double stent according to claim 11, **characterized in that** the flexible tongues (6) are formed on ring segments (7b) which are arranged adjacent to the peripheral ring segments (7a).

13. Double stent according to any one of claims 7 to 12, **characterized in that** the connecting webs (8) of the first inner stent (2) and second outer stent (3) are spaced apart with gaps between.

14. Double stent according to any one of the preceding claims, **characterized in that** the first membrane (4) and/or second membrane (5) consists of plastic material.

15. Double stent according to claim 14, **characterized in that** the first membrane (4) and/or the second membrane (5) consists of PTFE.

## Revendications

1. Double stent avec deux stents (2, 3) disposés de manière coaxiale, dans lequel une première membrane (4) est disposée entre un premier stent intérieur (2) et le second stent extérieur (3) et une deuxième membrane (5) est disposée sur le second stent (3), dans lequel les extrémités de membrane de la première membrane et de la deuxième membrane (4, 5) sont réunies sur les extrémités des stents (2, 3) et sont retournées sur le côté intérieur du premier stent (2) et y sont fixées, dans lequel le premier stent intérieur (2) est produit à partir d'un premier matériau et le second stent extérieur (3) est produit à partir d'un second matériau, **caractérisé en ce que** le second stent extérieur (3) est constitué d'un alliage de cobalt-chrome et le premier stent intérieur (2) est constitué d'un alliage à mémoire de forme et le premier stent intérieur (2) est plus long de 5 à 10 mm plus que le second stent extérieur (3) et présente dans son état final défini par mémoire de forme un diamètre plus grand de 1 à 6 mm que le second stent extérieur (3).

2. Double stent selon la revendication 1, **caractérisé en ce que** le premier stent intérieur (2) est constitué d'un alliage de nickel-titane.

3. Double stent selon la revendication 1 ou 2, **caractérisé en ce que** le premier stent intérieur (2) est plus long de 8 mm que le second stent extérieur (3).

4. Double stent selon l'une quelconque des revendications 1 à 3, **caractérisé en ce que** le premier stent intérieur (2) présente dans son état final défini par mémoire de forme un diamètre plus grand de 3 mm que le second stent extérieur (3).

5. Double stent selon l'une quelconque des revendications 1 à 4, **caractérisé en ce que** le premier stent intérieur (2) est pourvu d'éléments de fixation supplémentaires sur son extrémité proximale.

6. Double stent selon la revendication 5, **caractérisé en ce que** les éléments de fixation sont configurés sous la forme de barbe et/ou d'un élargissement en forme de trompette.

7. Double stent selon l'une quelconque des revendications précédentes, **caractérisé en ce qu'**au moins le premier stent intérieur (2) présente une multitude de segments annulaires (7, 7a, 7b) disposés côte à côte avec une structure en méandre, qui sont reliés entre eux par des entretoises de liaison (8, 9).

8. Double stent selon l'une quelconque des revendications précédentes, **caractérisé en ce que** les membranes (4, 5) sont fixées par collage, suture, soudure ou serrage.

9. Double stent selon l'une quelconque des revendications 1 à 8, **caractérisé en ce que** les membranes (4, 5) sont serrées dans des languettes à ressort (6) par des incisions dans des arcs de méandre du premier stent (2).

10. Double stent selon la revendication 9, **caractérisé en ce que** les languettes à ressort (6) pointent vers l'extérieur du stent.

11. Double stent selon la revendication 9 ou 10, **caractérisé en ce que** les languettes à ressort (6) sont disposées dans les zones de bord du premier stent (2).

12. Double stent selon la revendication 11, **caractérisé en ce que** les languettes à ressort (6) sont réalisées sur des segments annulaires (7b), qui sont disposés de manière adjacente à des segments annulaires (7a) côté bord.

13. Double stent selon l'une quelconque des revendications 7 à 12, **caractérisé en ce que** les entretoises de liaison (8) du premier stent intérieur (2) et du second stent extérieur (3) sont en quinconce.

14. Double stent selon l'une quelconque des revendications précédentes, **caractérisé en ce que** la première (4) et/ou la deuxième (5) membrane (4, 5) sont constituées de matière plastique.

15. Double stent selon la revendication 14, **caractérisé en ce que** la première (4) et/ou la deuxième (5) membrane (4, 5) sont constituées de PTFE.
